# EUROPEAN PATENT SPECIFICATION

(11) **EP 1 162 946 B1**
(45) Date of publication and mention of the grant of the patent: **16.12.2009**
(21) Application number: 99949008.9
(22) Date of filing: 18.10.1999
(51) Int. Cl.: A61K 9/00, A61K 9/68

(54) **COMPOSITION FOR MEDICATED CHEWING GUMS, PROCESS FOR MANUFACTURING THE SAME AND TABLETS SO OBTAINED**
ARZNEISTOFFHALTIGER KAUGUMMI, DESSEN HERSTELLUNGSVERFAHREN UND SO ERHALTENE TABLETTE
COMPOSITION POUR GOMMES A MACHER A USAGE MEDICAL, PROCEDE DE FABRICATION ET PRESENTATION SOUS FORME DE DRAGEES

(30) Priority: 22.03.1999 IT MI990571; 31.08.1999 US 387538
(43) Date of publication of application: 19.12.2001
(73) Proprietor: PIERRE FABRE MEDICAMENT, 92100 Boulogne-Billancourt (FR)
(72) Inventor: BADETTI, Rolando, I-20052 Monza (IT)
(74) Representative: Ahner, Francis
(86) International application number: PCT/EP1999/007917
(87) International publication number: WO 2000/056281

(56) References cited:
- EP-A- 0 177 368
- WO-A-84/03201
- WO-A-96/03111
- WO-A-97/12589
- GB-A- 934 596
- US-A- 4 885 175

## Description

The present invention relates to a composition comprising a therapeutically active principle for use in medicated chewing gums. The present invention relates particularly to a composition for medicated chewing gums comprising nicotine.

As the chewing gums have reached high popularity and compliance since the beginnings of this century, chewing gums containing a therapeutically active principle in particles dispersed in the gum and defined "medicated chewing gums" have been developed.

The problems associated with such gums are numerous such as the reproducibility of the release rate of the active principle from the gum, the stability and the masking of the unpleasant taste of the active substance and so on.

These problems were attempted to be solved first of all by using micro-encapsulation techniques which resulted not completely satisfactory because of the delay of the active principle release from the gum. Good results have been achieved by encapsulating such active principles in compounds suitable to include them such as the cyclodextrins and producing the gums with cold-pressing techniques. Such method serves to preserve the stability of the active principle and solve some of the above-noted technical problems.

None of these solutions allowed to solve another problem associated with chewing gums i.e. the feeling of irritation deriving from the contact of the therapeutically active principle with the mucous membrane (mucosas) of the oral cavity, a situation which is particularly annoying when the active principle has irritant properties, as in the case of nicotine.

An attempt at solving this problem is described in French patent 7 340 760 wherein a composition comprising a buffer system suitable to increase the physiological pH of the mouth mucous membrane, decreasing the irritant sensation of the mouth generated by the chewing gums comprising nicotine.

WO 96/03111 discloses a chewing gum in the form of tablets comprising an active ingredient in particle form, the particles possibly being microencapsulated or coated for delayed release of the active ingredient, each tablet being lacquered with a layer of lacquer based on a film-forming component selected from derivatives of cellulose and polyethylene glycols in a solvent selected from water, alcohols and acetone or a mixture thereof.

GB-A-934 596 discloses a slab chewing gum comprising a solid active material in powder form and dispersed in the gum base as discrete particles coated with sugar.

EP 0 177 368 discloses a chewing gum comprising a composite comprising an agglomerated mixture of a core material comprising a drug and a matrix, said matrix comprising 0.5 to 20% of fat soluble lecithine; 61 to 90% of an edible material having a melting point between 25 and 100°C chosen from a fatty acid having an iodine value of from 1 to 10 ; natural waxes; synthetic waxes and mixtures thereof and 0.5 to 20% of a glyceride.

US 4,885,175 discloses a chewing gum comprising a wax-coated delayed release ingredient.

Therefore it is an object of this invention to provide a chewing gum composition in order to attain a gradual and controlled release of nicotine which does not use a system altering the mouth pH, but which, however, avoids the throat irritation generated by nicotine while maintaining a high absorption thereof.

Another object of the present invention is to provide a chewing gum comprising nicotine which has high heat and humidity stability and high organoleptic properties.

It is still an object to provide an easy process which allows a chewing gum having high stability and organoleptic properties to be obtained.

It is still another object to provide a chewing gum comprising nicotine which allows the masking of the possible unpleasant taste generated by nicotine.

The objects cited above are achieved by providing a composition with the characteristics stated in claim 1 and obtained by the process stated in claim 14. The advantageous properties of the composition of the present invention are achieved providing for the characteristics stated in dependent claims.

The composition according to the invention is characterized by the fact that nicotine particles which are dispersed in the gum are coated by a mixture comprising a water insoluble element and a hydrosoluble element.

The invention is also directed to formation of the composition into a tablet of medicated chewing gum wherein said tablet is stable at 40°C and 75% humidity for 6 months.

The invention is also directed to a use of the composition of the present invention as in claim 18.

The coating mixture of the nicotine is such that the hydrosoluble element dissolves in contact with saliva during the chewing function, generating particular pathways for the exit of the active principle, whereas the insoluble element remains in the gum. In such a way nicotine comes out and contacts the mouth mucous membranes, whereas the coating mixture remains in the gum.

The composition of the invention will be detailed together with the preferred illustrative embodiments and the Figure, which is a plot of the release curve of the active principle of a tablet according to the invention obtained through HPLC (High Pressure Liquid Chromatography) analysis.

The insoluble element according to the invention is a plant hydrogenated oil, preferably hydrogenated castor oil.

The soluble element is a polyhydric alcohol preferably selected from the group consisting of sorbitol, sucrose, lactose, glucose, fructose, mannitol, xilitol, isomalt, more preferably sorbitol.

The active principle contained in the chewing gum according to the invention is nicotine. Preferably, nicotine in the form of nicotine polyacrylate in amounts between 0.1 and 20% by weight, preferably 1.1% by weight (11.11 mg of the total formulation). Such a composition comprising nicotine is suitable for the use in the treatment for giving up smoking.

If the amount of the active principle is defined as Z, then the insoluble element is between 1Z and 10Z and the soluble element between 2Z and 20Z. In other words, the insoluble element is present in the ratio of from 1 to 10:1 of the active principle, and the soluble element is present in the ratio of from 2 to 20:1 of the active principle. The composition according to the invention comprises as active principle nicotine polyacrylate in amounts of 1.11% by weight of the total composition, therefore the insoluble element will be between 1.11% and 11.1% by weight, preferably 6%, while the hydrosoluble element between 2.22% and 22.2%, preferably 10% by weight of the total composition.

As a matter of fact from the tests of *in vitro* release carried out for 30 minutes it was shown that if the insoluble element is below 1.1% then the release of nicotine increases at 5 and 10 minutes, remaining similar at higher times, while above 11.1% the release of nicotine decreases at 5 and 10 minutes and remains similar at subsequent times and that if the soluble element is below 2.22% the release is slower and incomplete, while above 22.2% the release is complete but quicker.

The composition of the invention preferably comprises nicotine as active principle, hydrogenated castor oil as insoluble element and sorbitol as hydrosoluble element, preferably in amounts of 1.1 %, 6% and 10% by weight of the total composition, respectively.

The composition of the invention comprises a gum base which is selected each time according to the health regulations of the countries where the product is consumed. For instance, the gum base in the present invention may comprise any suitable gum base material known in the art, including natural gum bases, such as chicle, jelutong, gutta percha and crown gum or synthetic gum materials such as butadiene-styrene rubber, isobutylene-isoprene copolymer, paraffin, petroleum waxes, polyethylene, polyisobutylene polyvinyl acetate, or blends thereof. In a preferred embodiment, the gum base comprises synthetic gum base materials.

The gum base composition may comprise from about 5 to 25%, preferably from about 10 to 18% by weight elastomers; from about 25 to 55% preferably from 38 to 48% by weight resins, from about 15 to 40% preferably from about 22 to 32% by weight plasticizers; from about 10 to 25%, preferably from about 13-16% by weight water insoluble adjuvants; and from about 0.05 to 0.1 % preferably about 0.1 % by weight food grade anti-oxidants.

The gum base for use in the invention may be prepared by cooling the gum to -30/-40°C and grinding it.

The composition according to the invention can also contain additives among which flavours, sweeteners, dehydration agents, pH stabilizers, inclusion agents, lubricants, compression adjuvants, etc can be mentioned.

Flavouring agents suitable for use in the invention include essential oils and synthetic flavours such as citrus oils, fruit essences, peppermint oil, spearmint oil, clove oil, oil of wintergreen, anise and the like. Artificial flavorants known to those skilled in the art are also contemplated for use in the invention.

Compression adjuvants may also be added. These compounds facilitate compression of the gum into tablets. Suitable compression adjuvants include silicon dioxide, magnesium stearate, behenic acid, talc and similar substances. Compression adjuvants are often essential to limit the tendency of the gum tablets to stick to the presses during manufacture.

The sweeteners according to the invention are preferably selected from the group consisting of acesulfame K, aspartame, saccharin, cyclamates, neoesperidine, maltol, ethylmaltol.

The pH of the composition will be between 5 and 10, preferably 7.5.

The chewing gum according to the invention is obtained with the process consisting of the following steps:
a) heating the insoluble plant hydrogenated oil until a complete solution is obtained;
b) mixing the soluble polyhydric alcohol with the nicotine;
c) pouring the mixture of step b) in the plant hydrogenated oil solution obtained at step a)
d) cooling the mixture so obtained and mixing with the gum base and the additives; and
e) pressing at a temperature not above room temperature.

Preferably the active principle is nicotine, the insoluble element is hydrogenated castor oil and the soluble element is sorbitol. Castor oil is preferably heated between 50 e 130°C, more preferably 90°C and the cooling of step d) is at between 1 and 10°C, particularly 5°C.

The process of the invention provides also for the addition of an inclusion substance in step b), e.g. β-cyclodextrin.

Examples of manufacturing the composition, formulations of the invention together with tests for determining its active principle release characteristics, its stability and its compliance now follow.

### Example 1

In a 11 beaker 60 g of hydrogenated castor oil were heated at about 90°C until a complete solution is obtained. Separately in a polyethylene bag 11.11 g of nicotine polyacrylate, 100 g of sorbitol and 50 g of β-cyclodextrin were mixed. The powder sieved through a sieve of 710 micron was added in the hydrogenated castor oil at 90-100°C. The solution was vigorously stirred in order to avoid the formation of lumps. The mixture was then left at a temperature of 5°C for 1 hour, thereafter it was sieved through the sieve of 710 micron. A granulate, which was mixed with 579.49 g of gum base, 64 g of Wintergreen flavour, 64 g of food sweet flavour, 1.3g of menthol, 3.2 of aspartame, 1.9 g of acesulfame K, 22.5 g of syloid 244 and 22.5 g of talc and, finally, with 20 g of magnesium stearate, is obtained. After careful mixing it was pressed in a Ronchi R18 type pressure machine so as to obtain chewable tablets of 1000 mg. The yield in tablets is above 95%.
The composition of the tablet so obtained is the following:

| | |
|---|---|
| Nicotine polyacrylate 18% | 11.11 mg |
| (equivalent to 2 mg of nicotine base) | |
| Gum base for chewing gum | 579.49 mg |
| Hydrogenated castor oil | 60 mg |
| Sorbitol | 100 mg |
| Wintergreen flavour | 64 mg |
| Food sweet flavour | 64 mg |
| Menthol | 1.3 mg |
| Aspartame | 3.2 mg |
| Acesulfame K | 1.9 mg |
| Syloid 244 | 22.5 mg |
| Talc | 22.5 mg |
| Magnesium stearate | 20 mg |
| β-cyclodextrin | 50 mg |
| **TOTAL** | **1000.0 mg** |

### IN VITRO RELEASE

A tablet of 1000 mg comprising 2 mg of nicotine was subjected to HPLC (High Pressure Liquid Chromatography) for determining in vitro release of the nicotine by following the procedure stated below.

A device "Water 820" supplied with a column Supelco C18 x 12.5 cm was used. A flow of 1.5 ml/min and wavelength of 254 nm were set. The conditioning of the column was carried out with a mobile phase consisting of ACCN, CH₃COOH, sodium laurylsulfate, sodium acetate and water; retention time was from 3.5 to 5.5 min; sample concentration was 0.04 mg/ml and standard concentration was 0.04 mg/ml in mobile phase.

50 ml of water and a tablet according to the invention were introduced in a suitable bag assuring that all air flows out of it. A chewing machine was operated at "low" speed. 1 ml was collected from the bag at 5, 10, 15, 20, 30 minutes and every time the collected sample was restored with water.

The collected samples were filtered by means of a syringe and 0.45 µ filter and injected on HPLC column which was conditioned as described above.

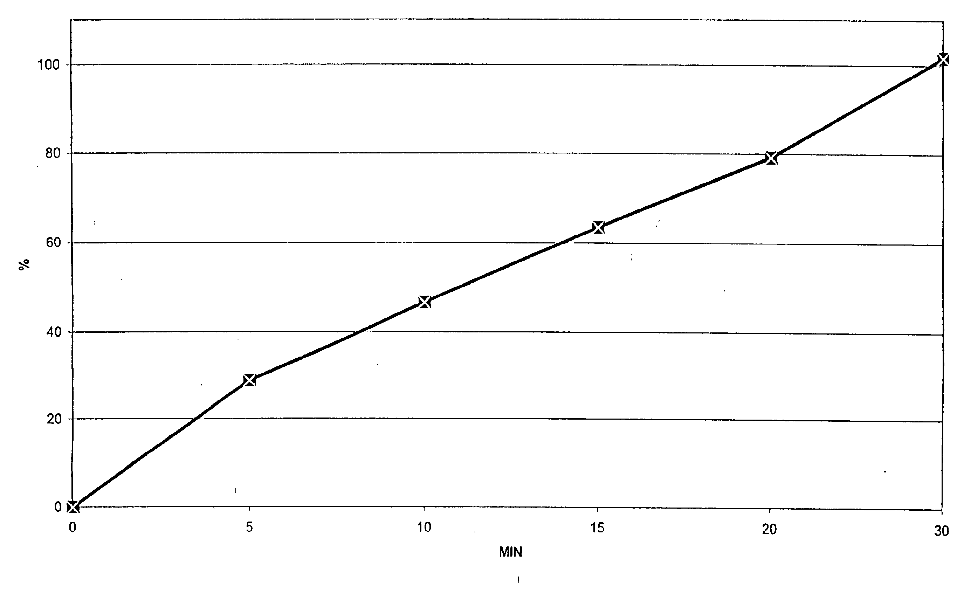

The release curve plotted in Figure 1 was obtained, from which it can be seen that the release is almost constant between 0 and 30 minutes reaching the complete release at 30 minutes.

### IN VIVO RELEASE

Three tablets according to the invention (batch V0018), each comprising 2 mg of nicotine were administered respectively to three adult subjects and the plasmatic levels of nicotine were determined by taking of blood samples. Such *in vivo* release tests were carried out by I.P.A.S. S.A of Ligornetto (Switzerland). The plasmatic levels obtained at times of taking blood samples are shown in the following Table 1.

**Table 1**

| **Time (min)** | **Conc. (ng/ml)** | **Conc. (ng/ml)** | **Conc. (ng/ml)** |
|---|---|---|---|
| | **Subject 1** | **Subject 2** | **Subject 3** |
| 0 | 0 | 0 | 0 |
| 10 | 3.98 | 2.14 | 3.32 |
| 20 | 3.63 | 2.2 | 3.39 |
| 30 | 3.51 | 2.81 | 3.28 |
| 40 | 4.09 | 2.74 | 3.61 |
| 50 | 2.71 | 2.88 | 3.96 |
| 60 | 3.94 | 2.74 | 2.16 |
| 75 | 3.52 | 3.07 | 3.4 |
| 90 | 2.85 | 2.95 | 3.21 |
| 105 | 2.13 | 3.05 | 2.77 |
| 120 | 2.03 | 2.45 | 2.41 |
| 150 | 1.53 | 1.65 | 2.01 |
| 180 | 1.08 | 1.69 | 1.89 |
| 210 | 0.918 | 1.54 | 1.45 |
| 240 | 0.991 | 1.08 | 1.25 |
| 360 | 0 | 0 | 0.582 |
| 480 | 0 | 0 | 0 |

From the concentration data the maximum concentration (Cmax), the time required to obtain it (Tmax) and the area under the curve (AUC) - which was drawn joining the points corresponding to times of taking blood samples - were therefore obtained for each of the tested subjects. (Table 2).

**Table 2**

| **Subject** | **Cmax (ng/ml)** | **Tmax (min)** | **AUC** |
|---|---|---|---|
| Subject 1 | 4.09 | 40 | 8.70 |
| Subject 2 | 3.07 | 75 | 8.60 |
| Subject 3 | 3.96 | 50 | 11.4 |

From the above data it is clear that nicotine reaches high plasmatic levels within 40-75 minutes confirming good bioavailability and strong absorption of the active principle when the composition of the invention is used.

### QUALITY ANALYSIS

One tablet according to the invention (Batch TF 599) comprising 2 mg of nicotine was subjected to quality control according to the specifications stated in Table 3. The results which led to approval of the tablets of the invention are shown in the last column of the table.

**Table 3**

| **TEST** | **SPECIFICATIONS** | **RESULTS** |
|---|---|---|
| Pharmaceutical form | Chewing gum | Approved |
| Form | Round | Approved |
| Colour | Beige | Approved |
| Flavour | Wintergreen | Approved |
| Length-Width | 19.5 x 11.5 mm | 19.5 x 11.5 mm |
| Height | 4.5-5.5 mm | 4.9 mm |
| Hardness | 5 - 15 Kp | 10 Kp |
| Medium weight | 950-1050 mg | 1005 mg |
| Water content | ≤ 2% | 0.59% |
| Nicotine content | 1.9 - 2.1 mg | 2.03 mg |
| | 95 - 105% | 101.5% |

### STABILITY CONTROL

The product of the invention comprising 2 mg of nicotine (batch: TF 599) was studied in order to determine the stability for 6 months at 40-75°C of humidity, packaged in blisters of the following materials: PVC, PVC + ALU, PVDC and PVDC + ALU. The tablets according to the invention resulted in conformity with chemical and physical controls and therefore stable in the studied conditions.

From the stability plan a stability for 12 months at 30°C - 60% of humidity and for 24 months at 25 - 60% of humidity was inferred.

### COMPARATIVE COMPLIANCE STUDY

A comparative study between a medicated chewing gum with nicotine according to the invention and a comparative formulation, which is commercially available as NICORETTE® manufactured by Pharmacia-Upjohn and does not contain the coating mixture according to the invention, was carried out. Six volunteers were periodically examined.

All the volunteers defined the taste of the formulation test as being better and, during the chewing, nobody felt irritation which was noticed for the comparative formulation.

## Claims

1. Composition for medicated chewing gum, **characterized by** the fact that it contains particles of nicotine as active principle dispersed in the gum, which are coated by a mixture consisting of a hydrosoluble polyhydric alcohol and a water insoluble plant hydrogenated oil, said coating allowing a gradual and controlled release of the active principle.

2. Composition according to claim 1 wherein the active principle is nicotine polyacrilate.

3. Composition according to anyone of claims 1 and 2, wherein the insoluble plant hydrogenated oil is hydrogenated castor oil.

4. Composition according to anyone of claims 1 to 3, wherein the hydrosoluble polyhydric alcohol is sorbitol.

5. Composition according to anyone of the preceding claims, wherein the nicotine content is between 0.1 and 20 % by weight, preferably 1.11 % of the total composition.

6. Composition according to anyone of the preceding claims, wherein if the amount of the nicotine is defined as Z, then the water insoluble plant hydrogenated oil is between 1Z and 10Z.

7. Composition according to anyone of the preceding claims, wherein if the amount of the nicotine is defined as Z, then the soluble polyhydric alcohol is between 2Z and 20Z.

8. Composition according to anyone of the preceding claims, wherein if the active principle is nicotine polyacrylate in amounts of 1.11 % by weight of the total composition, then the insoluble plant hydrogenated oil is between 1.11 % and 11.1 % by weight, preferably hydrogenated castor oil in amounts of 6.0 % by weight of the total composition.

9. Composition according to anyone of the preceding claims, wherein if the active principle is nicotine polyacrylate in amounts of 1.1 % by weight of the total composition, then the hydrosoluble polyhydric alcohol is between 2.22 % and 22.2 %, preferable sorbitol in amounts of 10 % by weight of the total composition.

10. composition according to anyone of the preceding claims comprising additives selected from the group consisting of flavours, sweeteners, dehydration agents, pH stabilizers, inclusion agents, lubricants.

11. Composition according to claim 10, wherein the flavour is Wintergreen.

12. Composition according to anyone of claims 10 and 11, wherein the sweeteners are selected from the group consisting of acesulfame K, aspartame, saccharin, cyclamates, neoesperidine, maltol, ethylmaltol.

13. Composition according to anyone of the preceding claims, wherein the pH of the composition is between 5 and 10, preferably 7.5.

14. Process for obtaining the medicated chewing gum having the composition according to anyone of claims 1-13, **characterized by** the following steps :
a) Heating the insoluble plant hydrogenated oil until a complete solution is obtained ;
b) Mixing the soluble polyhydric alcohol with the nicotine ;
c) Pouring the mixture of step b) in the plant hydrogenated oil solution obtained at step a);
d) Cooling the mixture so obtained and mixing with the gum base and the other additives, and ;
e) Pressing at a temperature not above room temperature.

15. process according to claim 14, wherein in step a) the insoluble plant hydrogenated oil is hydrogenated castor oil heated between 50°C and 130°C, more preferably at 90°C.

16. Process according to anyone of claims 14 and 15, wherein the cooling temperature in step d) is between 1°C and 10°C, particularly at 5°C.

17. Tablet of medicated chewing gum having the composition according to anyone of claims 1 to 13 and obtainable by the process according to anyone of claims 14 to 16, **characterized by** stability at temperature of 40°C and 75 % of humidity.

18. Use of the composition according to anyone of claims 1 to 13 for the preparation of a chewing gum tablet in the treatment for breaking smoking dependency.

## Patentansprüche

1. Zusammensetzung für einen medizinischen Kaugummi, **gekennzeichnet durch** die Tatsache, dass sie Nikotinteilchen als aktiven Grundbestandteil im Gummi verteilt enthält, welche **durch** ein Gemisch bestehend aus einem wasserlöslichen mehrwertigen Alkohol und einem wasserunlöslichen hydrierten Pflanzenöl beschichtet sind, wobei die Beschichtung eine stufenweise und kontrollierte Freisetzung des aktiven Grundbestandteils ermöglicht.

2. Zusammensetzung nach Anspruch 1, wobei es sich bei dem aktiven Grundbestandteil um Nikotin-Polyacrylat handelt.

3. Zusammensetzung nach einem der Ansprüche 1 und 2, wobei es sich bei dem unlöslichen hydrierten Pflanzenöl um hydriertes Castoröl handelt.

4. Zusammensetzung nach einem der Ansprüche 1 bis 3, wobei es sich bei dem wasserlöslichen mehrwertigen Alkohol um Sorbitol handelt.

5. Zusammensetzung nach einem der vorhergehenden Ansprüche, wobei der Nikotingehalt zwischen 0,1 und 20 Gew.-% und vorzugsweise 1,11 % der gesamten Zusammensetzung beträgt.

6. Zusammensetzung nach einem der vorhergehenden Ansprüche, wobei, wenn die Menge des Nikotins als Z definiert ist, das wasserunlösliche hydrierte Pflanzenöl dann zwischen 1Z und 10Z ist.

7. Zusammensetzung nach einem der vorhergehenden Ansprüche, wobei, wenn die Menge des Nikotins als Z definiert ist, der lösliche mehrwertige Alkohol dann zwischen 2Z und 20Z ist.

8. Zusammensetzung nach einem der vorhergehenden Ansprüche, wobei, wenn es sich bei dem aktiven Grundbestandteil um Nikotin-Polyacrylat in Mengen von 1,11 Gew.-% der gesamten Zusammensetzung handelt, handelt, das unlösliche hydrierte Pflanzenöl dann zwischen 1,11 Gew.-% und 11,1 Gew.-% und vorzugsweise hydriertes Castoröl in Mengen von 6,0 Gew.-% der gesamten Zusammensetzung ist.

9. Zusammensetzung nach einem der vorhergehenden Ansprüche, wobei, wenn es sich bei dem aktiven Grundbestandteil um Nikotin-Polyacrylat in Mengen von 1,1 Gew.-% der gesamten Zusammensetzung handelt, handelt, der wasserlösliche mehrwertige Alkohol dann zwischen 2,22 Gew.-% und 22,2 Gew.-% und vorzugsweise Sorbitol in Mengen von 10 Gew.-% der gesamten Zusammensetzung ist.

10. Zusammensetzung nach einem der vorhergehenden Ansprüche, umfassend Zusatzstoffe, die aus der Gruppe bestehend aus Geschmackstoffen, Süßungsmitteln, Dehydratisierungsmitteln, pH-Stabilisatoren, Einschlussmitteln und Gleitmitteln ausgewählt sind.

11. Zusammensetzung nach Anspruch 10, wobei es sich bei dem Geschmackstoff um Wintergrün handelt.

12. Zusammensetzung nach einem der Ansprüche 10 und 11, wobei die Süßungsmittel aus der Gruppe bestehend aus Acesulfame K, Aspartam, Saccharin, Cyclamaten, Neohesperidin, Maltol und Ethylmaltol ausgewählt sind.

13. Zusammensetzung nach einem der vorhergehenden Ansprüche, wobei der pH der Zusammensetzung zwischen 5 und 10 und vorzugsweise 7,5 beträgt.

14. Verfahren zum Erhalten des medizinischen Kaugummis mit der Zusammensetzung nach einem der Ansprüche 1 bis 13, **gekennzeichnet durch** die folgenden Schritte:
a) Erwärmen des unlöslichen hydrierten Pflanzenöls, bis eine vollständige Lösung erhalten wird;
b) Mischen des löslichen mehrwertigen Alkohols mit dem Nikotin;
c) Leeren des Gemisches von Schritt b) in die Lösung des hydrierten Pflanzenöls, die in Schritt a) erhalten wurde;
d) Abkühlen des so erhaltenen Gemisches und Mischen mit der Gummibasis und den anderen Zusatzstoffen, und
e) Pressen bei einer Temperatur nicht über Raumtemperatur.

15. Verfahren nach Anspruch 14, wobei es sich in Schritt a) bei dem unlöslichen hydrierten Pflanzenöl um hydriertes Castoröl handelt, das zwischen 50 °C und 130 °C und vorzugsweise bei 90 °C erwärmt wird.

16. Verfahren nach einem der Ansprüche 14 und 15, wobei die Abkühlungstemperatur in Schritt d) zwischen 1 °C und 10 °C und insbesondere bei 5 °C liegt.

17. Medizinische Kaugummitablette, aufweisend die Zusammensetzung nach einem der Ansprüche 1 bis 13 und erhältlich durch das Verfahren nach einem der Ansprüche 14 bis 16, **gekennzeichnet durch** Stabilität bei einer Temperatur von 40 °C und 75 % Feuchtigkeit.

18. Verwendung der Zusammensetzung nach einem der Ansprüche 1 bis 13 zur Herstellung einer Kaugummitablette bei der Behandlung zum Stoppen der Abhängigkeit vom Rauchen.

## Revendications

1. Composition pour un chewing-gum médicamenteux, **caractérisée par le fait qu'**elle contient des particules de nicotine comme principe actif dispersées dans la gomme, qui sont enrobées par un mélange constitué d'un alcool polyhydrique hydrosoluble et d'une huile hydrogénée végétale insoluble dans l'eau, ledit enrobage permettant une libération progressive et contrôlée du principe actif.

2. Composition selon la revendication 1, dans laquelle le principe actif est le poly(acrylate) de nicotine.

3. Composition selon l'une quelconque des revendications 1 et 2, dans laquelle l'huile hydrogénée végétale insoluble est de l'huile de ricin hydrogénée.

4. Composition selon l'une quelconque des revendications 1 à 3, dans laquelle l'alcool polyhydrique hydrosoluble est le sorbitol.

5. Composition selon l'une quelconque des revendications précédentes, dans laquelle la teneur en nicotine est comprise entre 0,1 et 20 % en poids, de préférence de 1,11 % de la composition totale.

6. Composition selon l'une quelconque des revendications précédentes, dans laquelle si la quantité de nicotine est définie par Z, alors l'huile hydrogénée végétale insoluble dans l'eau est comprise entre 1Z et 10Z.

7. Composition selon l'une quelconque des revendications précédentes, dans laquelle si la quantité de nicotine est définie par Z, alors l'alcool polyhydrique soluble est compris entre 2Z et 20Z.

8. Composition selon l'une quelconque des revendications précédentes, dans laquelle si le principe actif est du poly(acrylate) de nicotine en quantité de 1,11 % en poids de la composition totale, alors l'huile hydrogénée végétale insoluble est comprise entre 1,11 % et 11,1 % en poids, de préférence de l'huile de ricin hydrogénée en quantité de 6,0 % en poids de la composition totale.

9. Composition selon l'une quelconque des revendications précédentes, dans laquelle si le principe actif est du poly(acrylate) de nicotine en quantité de 1,1 % en poids de la composition totale, alors l'alcool polyhydrique hydrosoluble est compris entre 2,22 % et 22,2 %, de préférence du sorbitol en quantité de 10 % en poids de la composition totale.

10. Composition selon l'une quelconque des revendications précédentes, comprenant des additifs choisis dans le groupe consistant en les arômes, les édulcorants, les agents de déshydratation, les stabilisateurs de pH, les agents d'inclusion et les lubrifiants.

11. Composition selon la revendication 10, dans laquelle l'arôme est la gaulthérie.

12. Composition selon l'une quelconque des revendications 10 et 11, dans laquelle les édulcorants sont choisis dans le groupe consistant en l'acésulfame K, l'aspartame, la saccharine, les cyclamates, la néoespéridine, le maltol et l'éthylmaltol.

13. Composition selon l'une quelconque des revendications précédentes, dans laquelle le pH de la composition est compris entre 5 et 10, de préférence de 7,5.

14. Procédé d'obtention du chewing-gum médicamenteux ayant la composition selon l'une quelconque des revendications 1 à 13, **caractérisé par** les étapes suivantes consistant à :
a) chauffer l'huile hydrogénée végétale insoluble jusqu'à l'obtention d'une solution complète ;
b) mélanger l'alcool polyhydrique insoluble avec la nicotine ;
c) verser le mélange de l'étape b) dans la solution d'huile hydrogénée végétale obtenue à l'étape a) ;
d) refroidir le mélange ainsi obtenu et mélanger avec la gomme de base et les autres additifs ; et
e) presser à une température ne dépassant pas la température ambiante.

15. Procédé selon la revendication 14, dans lequel à l'étape a), l'huile hydrogénée végétale insoluble est de l'huile de ricin hydrogénée chauffée entre 50 °C et 130 °C, de manière davantage préférée à 90 °C.

16. Procédé selon l'une quelconque des revendications 14 et 15, dans lequel la température de refroidissement à l'étape d) est comprise entre 1 °C et 10 °C, de préférence à 5 °C.

17. Tablette de chewing-gum médicamenteux ayant la composition selon l'une quelconque des revendications 1 à 13, et susceptible d'être obtenue par le procédé selon l'une quelconque des revendications 14 à 16, **caractérisée par** une stabilité à une température de 40 °C et 75 % d'humidité.

18. Utilisation de la composition selon l'une quelconque des revendications 1 à 13, pour la préparation d'une tablette de chewing-gum dans le traitement destiné à interrompre la dépendance au tabagisme.
